(19) Europäisches Patentamt
European Patent Office
Office européen des brevets

(11) EP 1 414 829 B1

(12) # EUROPÄISCHE PATENTSCHRIFT

(45) Veröffentlichungstag und Bekanntmachung des Hinweises auf die Patenterteilung:
**18.10.2006 Patentblatt 2006/42**

(21) Anmeldenummer: **02767330.0**

(22) Anmeldetag: **06.08.2002**

(51) Int Cl.:
***C07F 5/00*** *(2006.01)* ***A61K 31/28*** *(2006.01)*
***A61P 35/00*** *(2006.01)*

(86) Internationale Anmeldenummer:
**PCT/EP2002/008766**

(87) Internationale Veröffentlichungsnummer:
**WO 2003/014128 (20.02.2003 Gazette 2003/08)**

(54) **TUMORHEMMENDE CERVERBINDUNGEN**
TUMOUR INHIBITING CERIUM COMPOUNDS
COMPOSES CERVER INHIBANT LES TUMEURS

(84) Benannte Vertragsstaaten:
**AT BE BG CH CY CZ DE DK EE ES FI FR GB GR IE IT LI LU MC NL PT SE SK TR**

(30) Priorität: **06.08.2001 DE 10138561**

(43) Veröffentlichungstag der Anmeldung:
**06.05.2004 Patentblatt 2004/19**

(73) Patentinhaber: **Faustus Forschungs Cie. Translational Cancer Research GmbH**
**04109 Leipzig (DE)**

(72) Erfinder: **KEPPLER, Bernhard**
**68766 Hockenheim (DE)**

(74) Vertreter: **Grünecker, Kinkeldey, Stockmair & Schwanhäusser Anwaltssozietät**
**Maximilianstrasse 58**
**80538 München (DE)**

(56) Entgegenhaltungen:
- **CHEMICAL ABSTRACTS, vol. 60, no. 12, 1964 Columbus, Ohio, US; abstract no. 14105g, HART, F.A. ET AL.: "complexes of 1,10-phenanthroline with lanthanide chlorides and thiocyanates" XP002225592 & J. INORG. NUCL. CHEM., Bd. 26, Nr. 4, 1964, Seiten 579-585,**
- **CHEMICAL ABSTRACTS, vol. 63, no. 7, 1965 Columbus, Ohio, US; abstract no. 7878e, HART, F.A. ET AL.: "lanthanide complexes. (III). Complexes of 2,2'-dipyridyl with lanthanide chlorides, thiocyanates, acetates, and nitrates" XP002225593 & J. INORG. NUCL. CHEM., Bd. 27, Nr. 8, 1965, Seiten 1825-1829,**

EP 1 414 829 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft Cerverbindungen sowie deren Verwendung als Arzneimittel zur Prophylaxe und/oder Behandlung von Krebserkrankungen.

Komplexe von 1,10-Phenanthrolin mit Lanthanchloriden und -Thiocyanaten werden offenbart in Chemical Abstracts, Bd. 60, Abstract No. 14105g.

Komplexe von 2,2'-Dipyridyl mit Lanthanchloriden, -Thiocyanaten, -Acetaten und-Nitraten werden beschrieben in Chemical Abstracts, Bd. 63, Abstract No. 7878e.

Aufgabe der vorliegenden Erfindung ist es, eine Verbindung zur Verfügung zu stellen, die eine hohe Wirksamkeit zur Behandlung von Krebserkrankungen aufweist.

Diese Aufgabe wird gelöst durch eine Verbindung der allgemeinen Formel (I)

$$R^{i\oplus}\frac{i}{n}Y^{n\ominus} \qquad (I),$$

worin R eine Gruppe der allgemeinen Formel (A) ist

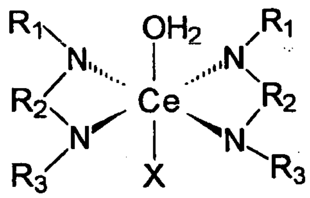

(A).

worin

$R_1$ und $R_3$ unabhängig voneinander ausgewählt sind aus der Gruppe bestehend aus $C_1$ - $C_{10}$ -Alkyl, $C_3$ - $C_6$-Cycloalkyl, $C_3$-$C_6$-Cycloalkenyl, $C_2$ - $C_{10}$-Alkenyl, $C_6$- $C_{14}$-Aryl, und einem Heterocyclus, die jeweils substituiert oder unsubstituiert sein können, und Wasserstoff;

$R_2$ $C_1$ - $C_6$ -Alkylen, $C_3$ - $C_6$ -Cycloalkylen, $C_3$ - $C_6$ -Cycloalkenylen, $C_2$ - $C_6$ -Alkenylen, $C_6$ - $C_{14}$-Arylen oder ein Heterocyclus, die jeweils substituiert oder unsubstituiert sein können, ist;

und $R_1$ und $R_2$, und/oder $R_2$ und $R_3$ einen Heterocyclus bilden können, der gegebenenfalls weitere Stickstoffatome enthalten kann;

X ein Halogen ist;

Y ein physiologisch verträgliches Anion ist; und

i und n unabhängig voneinander eine natürliche Zahl ≥ 1 sind, und

physiologisch verträglichen Additionssalzen,

mit der Maßgabe, dass die Verbindung der allgemeinen Formel (I) nicht

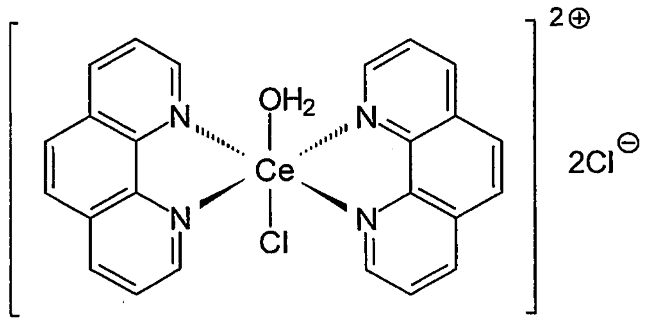

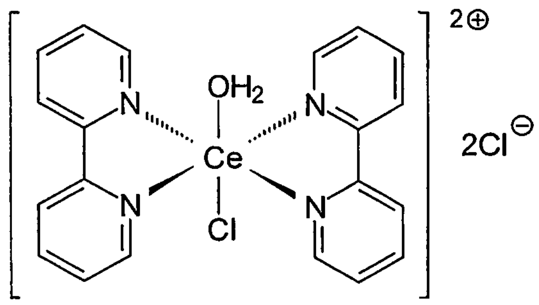

oder

ist.

In einer bevorzugten Ausführungsform sind $R_1$ und/oder $R_3$ $C_1$-$C_5$ Alkyl, insbesondere Methyl, Ethyl oder Propyl. Ferner sind $R_1$ und/oder $R_3$ bevorzugt Cyclobutyl, Cyclopropyl, Cyclobutenyl oder Cyclopropenyl und insbesondere Cyclopentyl, Cyclohexyl, Cyclopentenyl oder Cyclohexenyl, oder $C_2$-$C_5$-Alkenyl, insbesondere Ethenyl, Propenyl oder Butenyl. Weiterhin können $R_1$ und/oder $R_3$ Benzyl oder Pyridyl sein.

$R_2$ ist bevorzugt $C_1$-$C_5$ Alkylen, insbesondere Methylen, Ethylen oder Propylen. Ferner ist $R_2$ bevorzugt Cyclobutylen, Cyclopropylen, Cyclopentylen, Cyclohexylen, Cyclopentenylen oder Cyclohexenylen oder $C_2$-$C_5$-Alkenylen, insbesondere Ethenylen, Propenylen oder Butenylen. Weiterhin kann $R_2$ Benzylen oder Pyridylen sein.

$R_1$, $R_2$ und/oder $R_3$ können durch Hydroxyl, Amino, $-SO_3H$, Halogen, $C_1$-$C_4$-Alkyl, $C_2$-$C_4$-Alkenyl, $C_3$-$C_6$-Cycloalkyl, $C_3$-$C_6$-Cycloalkenyl, $C_1$-$C_6$-Aryl, $C_1$-$C_4$-Alkoxy, $C_1$-$C_4$-Alkoxy-$C_1$-$C_4$-alkylen, $C_1$-$C_4$-Alkylmercapto, $C_1$-$C_4$-Alkylmercapto-$C_1$-$C_4$-alkylen, Formyl, Carboxyl, $C_1$-$C_4$-Alkoxycarbonyl, $C_1$-$C_4$-Alkoxycarbonyl-$C_1$-$C_4$-alkylen, Di-$C_1$-$C_4$-alkylamino, Di-$C_1$-$C_4$-alkylamino-$C_1$-$C_4$-alkylen, Di-$C_1$-$C_4$-alkylaminocarbonyl, Di-$C_1$-$C_4$-alkylaminocarbonyl-$C_1$-$C_4$-alkylen, vorzugsweise Halogen und insbesondere Methyl, Ethyl oder Propyl substituiert sein, insbesondere wenn $R_1$ und $R_2$, und/oder $R_2$ und $R_3$ einen Heterocyclus bilden.

In einer weiteren bevorzugten Ausführungsform ist "i" die Zahl 2 und/oder "n" die Zahl 1.

Ferner ist X vorzugsweise Chlor.

Weiterhin ist Y in der allgemeinen Formel (I) bevorzugt ein Metallhalogen, ein Halogen, ein Pseudohalogen, $HCO_3$ oder R'COO, worin R' $C_1$ - $C_6$ -Alkyl, $C_2$ - $C_6$ -Alkenyl oder Aryl, die jeweils substituiert oder unsubstituiert sein können. Insbesondere ist Y Cl.

Organische oder anorganische Additionssalze können mit folgenden Anionen gebildet werden:

Chlorid, Bromid, Phosphat, Carbonat, Nitrat, Perchlorat, Sulfat, Citrat, Lactat, Tartrat, Maleat, Fumarat, Mandelat, Benzoat, Ascorbat, Cinnamat, Glycollat, Methansulfonat, Formiat, Malonat, Naphthalin-2-sulfonat, Salicylat und/oder Acetat.

Als mögliche Kationen können $H^+$, Natrium- und/oder Kalium-Kationen verwendet werden.

Ferner wird die Aufgabe der vorliegenden Erfindung durch ein Arzneimittel gelöst, enthaltend eine Verbindung der allgemeinen Formel (I)

$$R^{i\oplus}\frac{i}{n}\,Y^{n\ominus} \qquad \text{(I)},$$

worin R eine Gruppe der allgemeinen Formel (A) ist

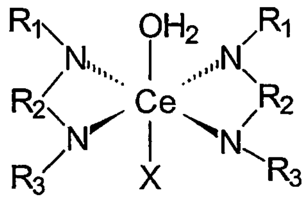

(A)

worin

$R_1$ und $R_3$ unabhängig voneinander ausgewählt sind aus der Gruppe bestehend aus $C_1$ - $C_{10}$ -Alkyl, $C_3$ - $C_6$-Cycloalkyl, $C_3$-$C_6$-Cycloalkenyl, $C_2$ - $C_{10}$-Alkenyl, $C_6$ - $C_{14}$-Aryl, und einem Heterocyclus, die jeweils substituiert oder unsubstituiert sein können, und Wasserstoff;

$R_2$ $C_1$ - $C_6$ -Alkylen, $C_3$- $C_6$-Cycloalkylen, $C_3$ - $C_6$-Cycloalkenylen, $C_2$ - $C_6$ -Alkenylen, $C_6$ - $C_{14}$-Arylen oder ein Heterocyclus, die jeweils substituiert oder unsubstituiert sein können, ist;

und $R_1$ und $R_2$, und/oder $R_2$ und $R_3$ einen Heterocyclus bilden können, der gegebenenfalls weitere Stickstoffatome enthalten kann;

X ein Halogen ist;

Y ein physiologisch verträgliches Anion ist; und

i und n unabhängig voneinander eine natürliche Zahl $\geq$ 1 sind, und

und physiologisch verträglichen Additionssalzen.

Für das Arzneimittel, enthaltend eine Verbindung der allgemeinen Formel (I) sind hinsichtlich der Gruppen $R_1$, $R_2$, $R_3$, X, Y, i und n dieselben Ausführungsformen bevorzugt wie oben für die erfindungsgemäße Verbindung dargestellt.
In bevorzugten Ausführungsformen kann die Gruppe R der allgemeinen Formel (A) aus

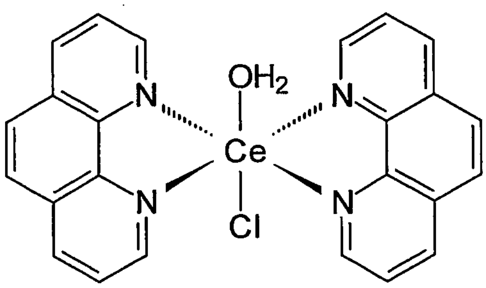

oder

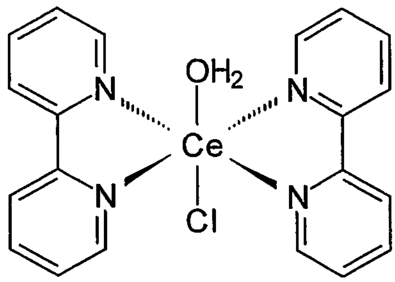

gewählt werden.
Die erfindungsgemäße Verbindung kann zur Prophylaxe und/oder Behandlung von Krebserkrankungen eingesetzt werden.
Diese Aufgabe wird in einer weiteren Ausführungsform gelöst durch eine Verbindung der allgemeinen Formel (II)

$$R_b^{+} Y_b^{-} \quad \text{(II)}$$

worin $R_b$ eine Gruppe der allgemeinen Formel (B) ist

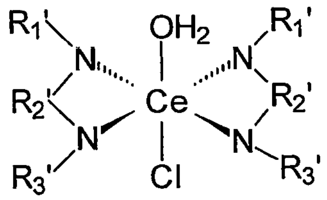

(B)

worin

$R_1$' und $R_3$ $C_1$ - $C_{10}$ -Alkyl, $C_3$- $C_6$-Cycloalkyl, $C_2$- $C_{10}$-Alkenyl, $C_6$ - $C_{14}$-Aryl, oder ein Heterocyclus, die jeweils substituiert oder unsubstituiert sein können, oder Wasserstoff ist;

$R_2$' $C_1$ - $C_6$ -Alkylen, $C_3$- $C_6$-Cycloalkylen, $C_2$ - $C_6$ -Alkenylen, $C_6$ - $C_{14}$-Arylen oder ein Heterocyclus, die jeweils substituiert oder unsubstituiert sein können, ist;

und $R_1$' und $R_2$', oder $R_2$' und $R_3$' einen Heterocyclus bilden können, der gegebenenfalls weitere Stickstoffatome enthalten kann;
und

$Y_b$ ein Metallhalogen, ein Halogen, ein Pseudohalogen, $HCO_3$ oder R'COO, worin R' $C_1$ - $C_6$ -Alkyl, $C_2$ - $C_6$ -Alkenyl oder Aryl, die jeweils substituiert oder unsubstituiert sein können, ist.

In bevorzugten Ausführungsformen kann die Gruppe $R_b$ der allgemeinen Formel (B) aus

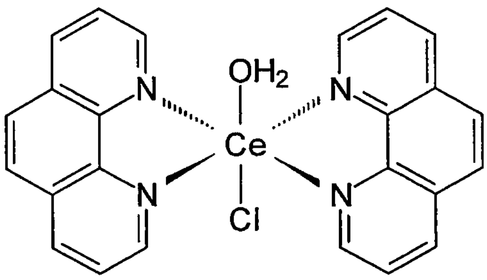

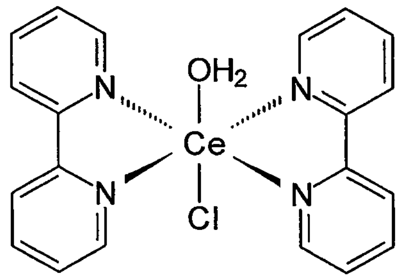

gewählt werden.

$R_1$' und $R_3$' sind bevorzugt $C_1$-$C_5$ Alkyl, insbesondere Methyl, Ethyl oder Propyl. Ferner sind $R_1$' und $R_3$' bevorzugt Cyclobutyl, Cyclopropyl oder $C_2$-$C_5$-Alkenyl, insbesondere Ethenyl, Propenyl oder Butenyl. Weiterhin können $R_1$' und $R_3$' Benzyl oder Pyridyl sein.

$R_1$' und $R_3$' können durch Methyl, Ethyl oder Propyl substituiert sein, insbesondere wenn $R_1$' und $R_2$', oder $R_2$' und $R_3$' einen Heterocyclus bilden.

$R_2$' ist bevorzugt $C_1$-$C_5$ Alkylen, insbesondere Methylen, Ethylen oder Propylen. Ferner ist $R_1$' und $R_3$' bevorzugt Cyclobutylen, Cyclopropylen oder $C_2$-$C_5$-Alkenylen, insbesondere Ethenylen, Propenylen oder Butenylen. Weiterhin kann $R_2$' Benzylen oder Pyridylen sein.

$R_2$' kann durch Methyl, Ethyl oder Propyl substituiert sein, insbesondere wenn $R_1$' und $R_2$' oder $R_2$' und $R_3$' einen Heterocyclus bilden.

Weiterhin ist $Y_b$ in der allgemeinen Formel (II) bevorzugt Cl.

Ferner wird die Aufgabe der vorliegenden Erfindung durch ein Arzneimittel gelöst, das die erfindungsgemäße Verbindung enthält. Die erfindungsgemäße Verbindung kann zur Prophylaxe und/oder Behandlung von Krebserkrankungen eingesetzt werden.

Im folgenden wird das Arzneimittel, enthaltend eine erfindungsgemäße Verbindung, genauer beschrieben.

Das erfindungsgemäße Arzneimittel wird vor allem intravenös, aber auch intramuskulär, intraperitoneal, subkutan oder peroral verabreicht. Auch eine äußerliche Applikation ist möglich. Bevorzugt ist die Verabreichung durch intravenöse Injektion oder intravenöse Infusion.

Das Arzneimittel wird nach an sich bekannten Verfahren hergestellt, wobei die erfindungsgemäße Verbindung als solche oder gegebenenfalls in Kombination mit geeigneten pharmazeutischen Trägerstoffen eingesetzt wird. Enthält

das erfindungsgemäße Arzneimittel neben dem Wirkstoff pharmazeutische Trägerstoffe, beträgt der Wirkstoffgehalt dieser Mischung 0,1 bis 99,5, vorzugsweise 0,5 bis 95 Gew.-% der Gesamtmischung.

Das erfindungsgemäße Arzneimittel kann in jeder geeigneten Formulierung angewandt werden unter der Voraussetzung, dass die Ausbildung bzw. Aufrechterhaltung von ausreichenden Wirkstoffpegeln gewährleistet ist. Das kann beispielsweise durch orale oder parenterale Gabe in geeigneten Dosen erreicht werden. Vorteilhafterweise liegt die pharmazeutische Zubereitung des Wirkstoffs in Form von Einheitsdosen vor, die auf die gewünschte Verabreichung abgestimmt sind. Eine Einheitsdosis kann zum Beispiel eine Tablette, ein Dragee, eine Kapsel, ein Suppositorium oder eine gemessene Volumenmenge eines Pulvers, eines Granulates, einer Lösung, einer Emulsion oder einer Suspension sein.

Unter "Einheitsdosis" im Sinne der vorliegenden Erfindung wird eine physikalisch bestimmte Einheit verstanden, die eine individuelle Menge des aktiven Bestandteils in Kombination mit einem pharmazeutischen Trägerstoff enthält und deren Wirkstoffgehalt einem Bruchteil oder Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel der Einheitsdosis benötigt wird, ist die Einheitsdosis vorteilhafterweise teilbar, z.B. in Form einer Tablette mit Bruchkerbe.

Die erfindungsgemäßen Arzneimittel können, wenn sie in Einheitsdosen vorliegen und für Applikationen z.B. am Menschen bestimmt sind, etwa 0,1 bis 500 mg, bevorzugt 10 bis 200 mg und insbesondere 50 bis 150 mg Wirkstoff enthalten.

Im allgemeinen werden in der Humanmedizin der oder die Wirkstoffe in einer Tagesdosis von 0,1 bis 5, vorzugsweise 1 bis 3 mg/kg Körpergewicht, gegebenenfalls in Form mehrerer, vorzugsweise 1 bis 3 Einzelgaben zur Erzielung der gewünschten Ergebnisse verabreicht. Eine Einzelgabe enthält den oder die Wirkstoffe in Mengen von 0,1 bis 5, vorzugsweise 1 bis 3 mg/kg Körpergewicht. Bei einer oralen Behandlung können ähnliche Dosierungen zur Anwendung kommen.

Die therapeutische Verabreichung des erfindungsgemäßen Arzneimittels kann 1 bis 4 mal am Tage zu festgelegten oder variierenden Zeitpunkten erfolgen, z.B. jeweils vor den Mahlzeiten und/oder am Abend. Es kann jedoch erforderlich sein, von den genannten Dosierungen abzuweichen, und zwar in Abhängigkeit von der Art, dem Körpergewicht und dem Alter der zu behandelnden Individuen, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Es kann sich auch als zweckmäßig erweisen, die Arzneimittel nur einmalig oder im Abstand von mehreren Tagen zu verabreichen.

Die Festlegung der erforderlichen optimalen Dosierung und Applikationsart der Wirkstoffe kann durch jeden Fachmann aufgrund seines Fachwissens erfolgen.

Die erfindungsgemäßen Arzneimittel bestehen in der Regel aus den erfindungsgemäßen Verbindungen und nichttoxischen, pharmazeutisch verträglichen Arzneimittelträgern, die als Zumischung oder Verdünnungsmittel, beispielsweise in fester, halbfester oder flüssiger Form oder als Umhüllungsmittel, beispielsweise in Form einer Kapsel, eines Tablettenüberzugs, eines Beutels oder eines anderen Behältnisses für den therapeutisch aktiven Bestandteil in Anwendung kommen. Ein Trägerstoff kann z.B. als Vermittler für die Arzneimittelaufnahme durch den Körper, als Formulierungshilfsmittel, als Süßungsmittel, als Geschmackskorrigens, als Farbstoff oder als Konservierungsmittel dienen.

Zur oralen Anwendung können z.B. Tabletten Dragees, harte und weiche Kapseln, z.B. aus Gelatine, dispergierbare Pulver, Granulate, wässrige und ölige Suspensionen, Emulsionen, Lösungen oder Sirupe kommen.

Tabletten können inerte Verdünnungsmittel, z.B. Calciumcarbonat, Calciumphosphat, Natriumphosphat oder Laktose; Granulierungs- und Verteilungsmittel, z.B. Maisstärke oder Alginate; Bindemittel, z.B. Stärke, Gelatine oder Akaziengummi; und Gleitmittel, z.B. Aluminium- oder Magnesiumstearat, Talkum oder Silikonöl, enthalten. Sie können zusätzlich mit einem Überzug versehen sein, der auch so beschaffen sein kann, dass er eine verzögerte Auflösung und Resorption der Arzneimittelzubereitung im Gastrointestinaltrakt bewirkt, so dass z.B. eine bessere Verträglichkeit, Protahierung oder Retardierung erreicht wird. Gelatinekapseln können den Arzneistoff vermischt mit einem festen, z.B. Calciumcarbonat oder Kaolin, oder einem öligen, z.B. Oliven-, Erdnuss-, oder Paraffinöl, Verdünnungsmittel enthalten.

Wässrige Suspensionen können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycatanol, Polyoxyethylensorbitolmonooleat oder Lecithin; Konservierungsmittel, z.B. Methyl- oder Propylhydroxybenzoate; Geschmacksmittel; Süßungsmittel, z.B. Saccharose, Lactose, Natriumcyclamat, Dextrose, Invertzuckersirup, enthalten.

Ölige Suspensionen können z.B. Erdnuss-, Oliven-, Sesam-, Kokos- oder Paraffinöl und Verdickungsmittel, wie z.B. Bienenwachs, Hartparaffin oder Cetylalkohol, enthalten; ferner Süßungsmittel, Geschmacksmittel und Antioxidantien.

In Wasser dispergierbare Pulver und Granulate können die erfindungsgemäße Verbindung in Mischung mit

Dispergier-, Benetzungs- und Suspendiermitteln, z.B. den oben genannten, sowie mit Süßungsmitteln, Geschmacksmitteln und Farbstoffen enthalten.

Emulsionen können z.B. Oliven-, Erdnuss-, oder Paraffinöl neben Emulgiermitteln, wie z.B. Akaziengummi, Traganthgummi, Phosphatiden, Sorbitanmonooleat, Polyoxyethylensorbitanmonooleat, und Süßungs- und Geschmacksmittel enthalten.

Wässrige Lösungen können Konservierungsmittel, z.B. Methyl- oder Propylhydroxybenzoate; Verdickungsmittel; Geschmacksmittel; Süßungsmittel, z.B. Saccharose, Laktose, Natriumcyclamat, Dextrose, Invertzuckersirup, sowie Geschmacksmittel und Farbstoffe enthalten.

Zur parenteralen Anwendung der Arzneistoffe dienen steril injizierbare, wässrige Lösungen, isotonische Salzlösungen oder sonstige Lösungen.

Die folgenden Beispiele erläutern die Erfindung.

**Beispiel 1**

**Synthese von *trans*-[Aquachlorobis(1,10-phenantrolin)cer(III)]-dichlorid**

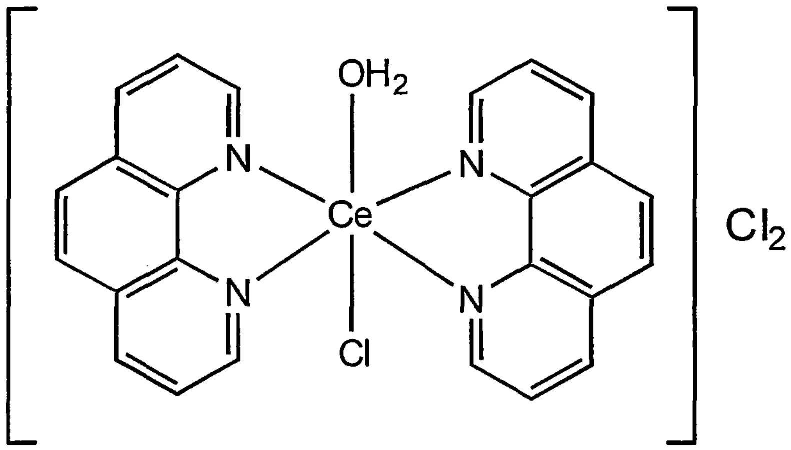

Die Herstellung von *trans*-[Aquachlorobis(1,10-phenantrolin)cer(III)]-dichlorid (KP 776) erfolgt durch Zugabe von Certrichloridhexahydrat ($CeCl_3 \cdot 6\ H_2O$) als 0,05 M ethanolische Lösung zu 1,10-Phenantrolinmonohydrat als 0,16 M ethanolische Lösung in einem molaren Verhältnis von 1 : 4. Das ausfallende Produkt wird nach 24 Stunden abfiltriert, mehrmals mit Ethanol gewaschen und im Vakuum bei Raumtemperatur getrocknet.

**Beispiel 2**

**Tumorhemmende Aktivität von trans-[Aquachlorobis(1,10-phenantrolin)cer(III)]-dichlorid**

Erste *in vitro*-Testungen wurden am National Cancer Institute (Bethesda, MD, USA) durchgeführt. Im 48 h-Sulforhodamin B-Assay an über 50 humanen Tumorzellinien wurde eine gute Wirksamkeit mit folgenden Kennwerten festgestellt:

| | | |
|---|---|---|
| mittlere $GI_{50}$: | 0,98 μmol/l | 0,61 μg/ml |
| mittlere TGI: | 15,1 μmol/l | 9,46 μg/ml |
| mittlere $LC_{50}$: | 63,1 μmol/l | 39,4 μg/ml |

Hierbei zeigte sich eine gewisse Selektivität für malignes Melanom. Überdurchschnittliche Aktivitäten wurden ferner an einzelnen Ovarial-, Nierzell- sowie kleinzelligen und nicht-kleinzelligen Bronchialkarzinom-Zellinien beobachtet.

Im Propidiumiodid-Assay an 13 humanen Tumorxenografts und 10 humanen Tumorzellinien zeigte sich ebenfalls eine gute tumorhemmende Aktivität mit folgenden Kenndaten:

| | | |
|---|---|---|
| mittlere $IC_{50}$: | 1,92 µmol/l | 1,20 µg/ml |
| mittlere $IC_{70}$: | 3,60 µmol/l | 2,25 µg/ml |
| mittlere $IC_{90}$: | 11,6 µmol/l | 7,27 µg/ml |

Auch hier wurde die stärkste Wirksamkeit an einem Melanom(-Xenograft) festgestellt.

## Patentansprüche

**1.** Verbindung der allgemeinen Formel (I)

$$R^{i\oplus} \frac{i}{n} Y^{n\ominus} \qquad \text{(I),}$$

worin R eine Gruppe der allgemeinen Formel (A) ist

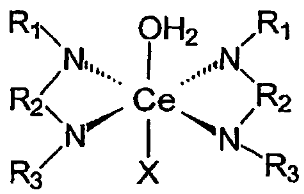

(A),

worin

$R_1$ und $R_3$ unabhängig voneinander ausgewählt sind aus der Gruppe bestehend aus $C_1$ - $C_{10}$ -Alkyl, $C_3$ - $C_6$-Cycloalkyl, $C_3$-$C_6$-Cycloalkenyl, $C_{2-}C_{10}$-Alkenyl, $C_6$ - $C_{14}$-Aryl, und einem Heterocyclus, die jeweils substituiert oder unsubstituiert sein können, und Wasserstoff;
$R_2$ $C_1$ - $C_6$ -Alkylen, $C_3$ - $C_6$-Cycloalkylen, $C_3$ - $C_6$-Cycloalkenylen, $C_2$ - $C_6$ - Alkenylen, $C_6$ - $C_{14}$-Arylen oder ein Heterocyclus, die jeweils substituiert oder unsubstituiert sein können, ist;
und $R_1$ und $R_2$, und/oder $R_2$ und $R_3$ einen Heterocyclus bilden können, der gegebenenfalls weitere Stickstoffatome enthalten kann;
X ein Halogen ist;
Y ein physiologisch verträgliches Anion ist; und
i und n unabhängig voneinander eine natürliche Zahl ≥ 1 sind, und
physiologisch verträglichen Additionssalzen,
mit der Maßgabe, dass die Verbindung der allgemeinen Formel (I) nicht

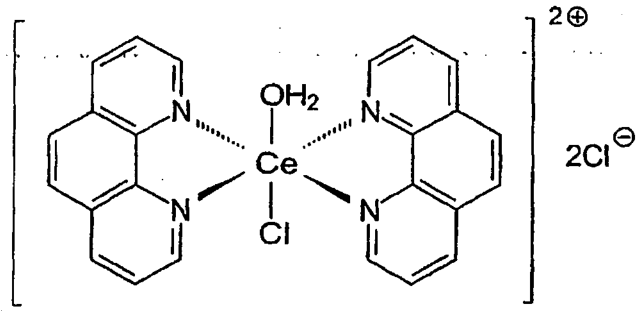

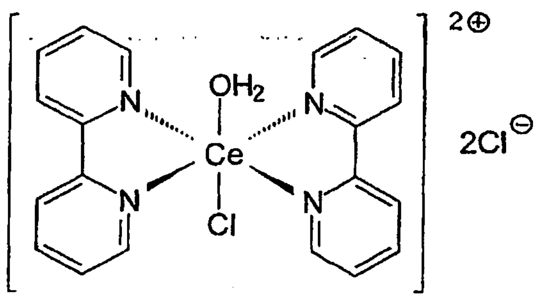

oder

ist.

2. Verbindung nach Anspruch 1, worin Y in der allgemeinen Formel (I) Cl ist.

3. Arzneimittel, enthaltend eine Verbindung der allgemeinen Formel (I)

$$R^{i\oplus} \frac{i}{n} Y^{n\ominus} \quad \text{(I)},$$

worin R eine Gruppe der allgemeinen Formel (A) ist

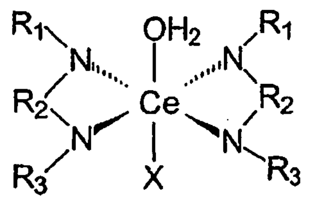

(A),

worin

$R_1$ und $R_3$ unabhängig voneinander ausgewählt sind aus der Gruppe bestehend aus $C_1$ - $C_{10}$ -Alkyl, $C_3$ - $C_6$-Cycloalkyl, $C_3$-$C_6$-Cycloalkenyl, $C_2$ - $C_{10}$-Alkenyl, $C_6$ - $C_{14}$-Aryl, und einem Heterocyclus, die jeweils substituiert oder unsubstituiert sein können, und Wasserstoff;
$R_2$ $C_1$ - $C_6$ -Alkylen, $C_3$ - $C_6$ -Cycloalkylen, $C_3$ - $C_6$ -Cycloalkenylen, $C_2$ - $C_6$ - Alkenylen, $C_6$ - $C_{14}$-Arylen oder ein Heterocyclus, die jeweils substituiert oder unsubstituiert sein können, ist;
und $R_1$ und $R_2$, und/oder $R_2$ und $R_3$ einen Heterocyclus bilden können, der gegebenenfalls weitere Stickstoffatome enthalten kann;
X ein Halogen ist;
Y ein physiologisch verträgliches Anion ist; und
i und n unabhängig voneinander eine natürliche Zahl ≥ 1 sind, und
und physiologisch verträglichen Additionssalzen.

4. Arzneimittel nach Anspruch 3, wobei die Gruppe R der Verbindung der allgemeinen Formel (I)

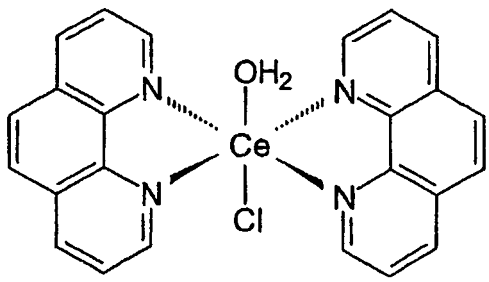

ist.

5. Arzneimittel nach Anspruch 3, wobei die Gruppe R der Verbindung der allgemeinen Formel (I)

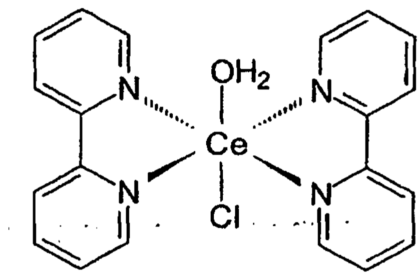

ist.

6. Verwendung einer Verbindung der allgemeinen Formel (I) nach Anspruch 1 oder 2 oder einer Verbindung der allgemeinen Formel (I) wie in einem der Ansprüche 3 bis 5 dargestellt zur Herstellung eines Arzneimittels zur Prophylaxe und/oder Behandlung von Krebserkrankungen.

**Claims**

1. Compound of the general formula (I)

$$R^{i\oplus}\frac{i}{n}Y^{n\ominus} \qquad \text{(I)},$$

where R is a group of the general formula (A)

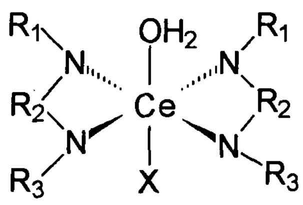

(A),

where

$R_1$ and $R_3$ are selected independently of one another from the group consisting of $C_1$-$C_{10}$-alkyl, $C_3$-$C_6$-cycloalkyl, $C_3$-$C_6$-cycloalkenyl, $C_2$-$C_{10}$-alkenyl, $C_6$-$C_{14}$-aryl and a heterocycle, which can in each case be substituted or

unsubstituted, and hydrogen;
$R_2$ is $C_1$-$C_6$-alkylene, $C_3$-$C_6$-cycloalkylene, $C_3$-$C_6$-cycloalkenylene, $C_2$-$C_6$-alkenylene, $C_6$-$C_{14}$-arylene or a heterocycle, which can in each case be substituted or unsubstituted;
and $R_1$ and $R_2$ and / or $R_2$ and $R_3$ can form a heterocycle which, where applicable, can contain further nitrogen atoms;
X is a halogen;
Y is a physiologically acceptable anion; and
i and n are independent of one another and are natural numbers ≥ 1, and
physiologically acceptable addition salts,
provided that the compound of the general formula (I) is not:

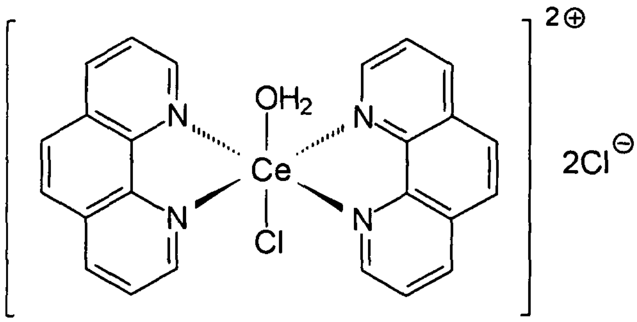

or

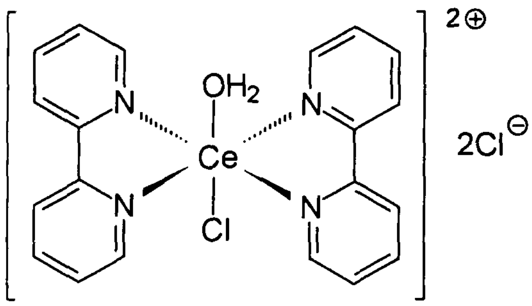

.

2. Compound according to Claim 1, where Y in the general formula (I) is Cl.

3. Medicament, containing a compound of the general formula (I)

$$R^{i\oplus} \frac{i}{n} Y^{n\ominus} \quad (I),$$

where R is a group of the general formula (A)

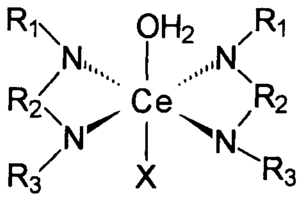

(A),

where

$R_1$ and $R_3$ are selected independently of one another from the group consisting of $C_{1-}C_{10}$-alkyl, $C_3$-$C_6$-cycloalkyl, $C_3$-$C_6$-cycloalkenyl, $C_2$-$C_{10}$-alkenyl, $C_6$-$C_{14}$-aryl and a heterocycle, which can in each case be substituted or unsubstituted, and hydrogen;
$R_2$ is $C_1$-$C_6$-alkylene, $C_3$-$C_6$-cycloalkylene, $C_3$-$C_6$-cycloalkenylene, $C_2$-$C_6$-alkenylene, $C_6$-$C_{14}$-arylene or a heterocycle, which can in each case be substituted or unsubstituted;
and $R_1$ and $R_2$ and / or $R_2$ and $R_3$ can form a heterocycle which, where applicable, can contain further nitrogen atoms;
X is a halogen;
Y is a physiologically acceptable anion; and
i and n are independent of one another and are natural numbers ≥ 1, and
physiologically acceptable addition salts.

**4.** Medicament according to Claim 3, wherein the group R of the compound of the general formula (I) is:

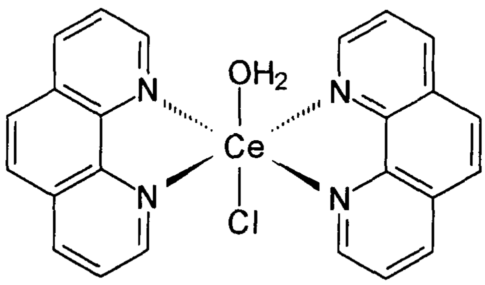

**5.** Medicament according to Claim 3, wherein the group R of the compound of the general formula (I) is:

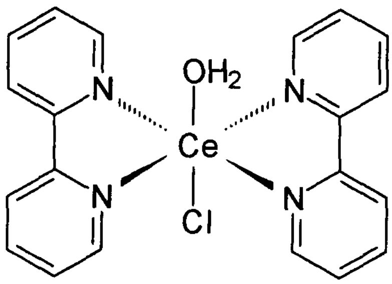

**6.** Use of a compound of the general formula (I) according to Claim 1 or 2 or a compound of the general formula (I) as presented in one of the Claims 3 to 5 for the preparation of a medicament for the prophylaxis and / or treatment of cancer diseases.

## Revendications

**1.** Composé de formule générale (I) :

$$R^{i\oplus}\frac{i}{n}Y^{n\ominus} \qquad \text{(I)},$$

dans laquelle R est un groupe de formule générale (A) :

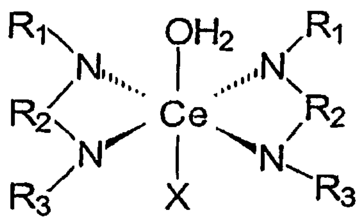

(A),

dans laquelle

$R_1$ et $R_3$ sont choisis, indépendamment l'un de l'autre, dans le groupe constitué par alkyle en $C_1$-$C_{10}$, cycloalkyle en $C_3$-$C_6$, cycloalcényle en $C_3$-$C_6$, alcényle en $C_2$-$C_{10}$, aryle en $C_6$-$C_{14}$, et un hétérocycle, qui peuvent dans chaque cas être substitués ou non substitués, et hydrogène ;
$R_2$ est alkylène en $C_1$-$C_6$, cycloalkylène en $C_3$-$C_6$, cycloalcénylène en $C_3$-$C_6$, alcénylène en $C_2$-$C_6$, arylène en $C_6$-$C_{14}$, ou un hétérocycle, qui peut dans chaque cas être substitué ou non substitué ; et
$R_1$ et $R_2$ et/ou $R_2$ et $R_3$ peuvent former un hétérocycle qui peut éventuellement contenir d'autres atomes d'azote ;
X est un halogène ;
Y est un anion physiologiquement compatible ; et
i et n sont, indépendamment l'un de l'autre, des nombres naturels ≥ 1 ; et
les sels d'addition physiologiquement compatibles,
avec la réserve que le composé de formule générale (I) n'est pas :

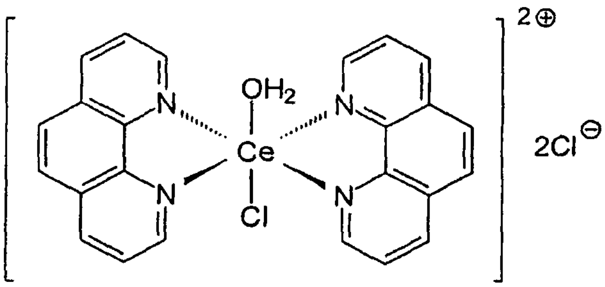

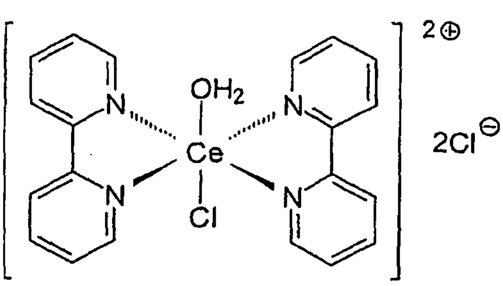

ou

**2.** Composé selon la revendication 1, dans lequel Y, dans la formule générale (I), est Cl.

**3.** Médicament contenant un composé de formule générale (I)

$$R^{i\oplus}\frac{i}{n}Y^{n\ominus} \qquad \text{(I)},$$

dans laquelle R est un groupe de formule générale (A) :

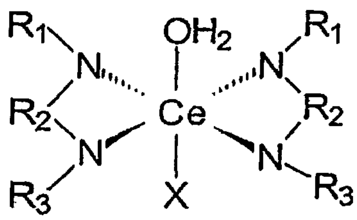

(A),

dans laquelle

$R_1$ et $R_3$ sont choisis, indépendamment l'un de l'autre, dans le groupe constitué par alkyle en $C_1$-$C_{10}$, cycloalkyle en $C_3$-$C_6$, cycloalcényle en $C_3$-$C_6$, alcényle en $C_2$-$C_{10}$, aryle en $C_6$-$C_{14}$, et un hétérocycle, qui peuvent dans chaque cas être substitués ou non substitués, et hydrogène ;
$R_2$ est alkylène en $C_1$-$C_6$, cycloalkylène en $C_3$-$C_6$, cycloalcénylène en $C_3$-$C_6$, alcénylène en $C_2$-$C_6$, arylène en $C_6$-$C_{14}$, ou un hétérocycle, qui peut dans chaque cas être substitué ou non substitué ; et
$R_1$ et $R_2$ et/ou $R_2$ et $R_3$ peuvent former un hétérocycle qui peut éventuellement contenir d'autres atomes d'azote ;
X est un halogène ;
Y est un anion physiologiquement compatible ; et
i et n sont, indépendamment l'un de l'autre, des nombres naturels ≥ 1 ; et
les sels d'addition physiologiquement compatibles.

**4.** Médicament selon la revendication 3, dans lequel R, dans la formule générale (I), est :

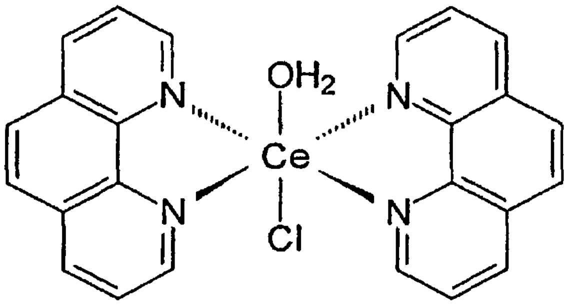

**5.** Médicament selon la revendication 3, dans lequel R, dans la formule générale (I), est :

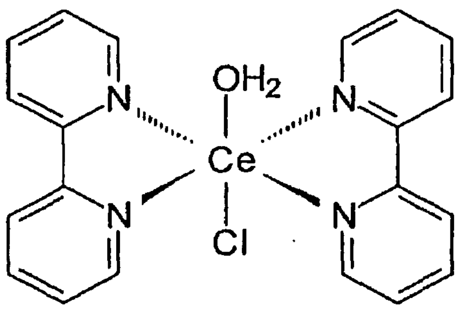

**6.** Utilisation d'un composé de formule générale (I) selon la revendication 1 ou 2 ou d'un composé de formule générale (I) tel que présenté dans l'une des revendications 3 à 5, pour la fabrication d'un médicament pour la prophylaxie et/ou le traitement de maladies cancéreuses.